# EUROPEAN PATENT SPECIFICATION

(11) **EP 0 966 286 B1**
(45) Date of publication and mention of the grant of the patent: **30.07.2003**
(21) Application number: 98901665.4
(22) Date of filing: 06.01.1998
(51) Int. Cl.: A61K 31/55, A61K 31/41, A61K 31/195, A61K 31/40, A61K 31/485, A61P 25/36

(54) **ATTENUATION OF OPIOID TOLERANCE BY INHIBITING INDUCIBLE NITRIC OXIDE SYNTHASE PATHWAYS IN THE TREATMENT OF PAIN**
VERBESSERUNG DER OPIOID-TOLERANZ DURCH HEMMUNG DER INDUZIERBAREN STICKSTOFFOXID-SYNTHASE ZUR BEHANDLUNG VON SCHMERZEN
ATTENUATION DE LA TOLERANCE AUX OPIOIDES PAR INHIBITION DES MECANISMES D'ACTION DE LA MONOXYDE D'AZOTE SYNTHETASE INDUCTIBLE, DANS LE TRAITEMENT DE LA DOULEUR

(30) Priority: 09.01.1997 US 781814
(43) Date of publication of application: 29.12.1999
(73) Proprietor: G.D. SEARLE & CO., Chicago, IL 60680-5110 (US)
(72) Inventor: SALVEMINI, Daniela, Ballwin, MI 63011 (US)
(74) Representative: Strych, Werner Dr.
(86) International application number: US9800006
(87) International publication number: WO98030220

(56) References cited:
- EP-A- 0 713 704
- WO-A-93/05775
- WO-A-95/11014
- WO-A-95/24382
- WO-A-96/18616
- WO-A-96/19440
- WO-A-96/27386
- WO-A-96/35677
- US-A- 5 256 669
- US-A- 5 321 012
- US-A- 5 502 058

## Description

### Field of the Invention

The present invention is directed to the use of inducible nitric oxide (iNOS) inhibitors in preventing and/or reversing tolerance that is seen upon prolonged usage of opioids in the clinical management of moderate to severe pain.

### Background of the Invention

The major opiate drugs, such as morphine, are widely used as analgesics in the clinical management of severe chronic pain. These drugs act on pain perception within the central nervous system in the clinical management of pain originating in the viscera or arising from severe injuries, burns, and neoplasms. Major opiate drugs that are used therapeutically to relieve severe pain seem to produce most of their analgesic actions through interactions with the µ type receptor.

Tolerance to the analgesic effects of opioids occurs upon prolonged usage. Well documented clinical example of this is seen in cancer patients. The net result of tolerance is decreased pain relief, increased side effects, and decreased clinical efficacy. Narcotic-induced hyperalgesia (NIH) may also develop in association with the tolerance of the opioid. This limits the clinical usefulness of these opiates in patients who require long term use of this opiate to relieve pain.

For instance, morphine is administered to a patient to relieve severe cancer pain. As the disease progresses, the intensity of the pain increases, and the morphine dosage increases. Tolerance develops and there is a decrease in analgesic effects. Thus the morphine dosage increases. Side effects develop including respiratory depression, vomiting, constipation, confusion, and immune system depression. There is an overall decrease in clinical efficacy

There has been no adequate strategy to overcome clinical development of tolerance and hence to provide an appropriate approach for the treatment of severe and chronic pain. One strategy used in the clinical management of opioid analgesic tolerance involves switching the type of opioid used on the assumption that receptor profiles of the commonly used opioid are dissimilar enough to reduce cross tolerance. Unfortunately the outcome of this action is not very satisfactory.

The mechanisms underlying opiate tolerance is poorly understood but it is known to involve the NMDA receptor since the NMDA receptor antagonist MK-801 has been shown in rats to prevent morphine tolerance. (Trujillo 1991). NMDA stimulates nitric oxide synthase (NOS) the enzyme responsible for the biosynthesis of nitric oxide (NO). Three isoforms of NOS have been identified to date. Two are constitutively expressed and are in general responsible for the beneficial effects described so far to NO. These are the endothelial and neuronal isoforms of NOS (eNOS and nNOS). The third isoform is inducible (iNOS) and accounts for the detrimental effects that have been observed for NO in several pathological conditions. Induction of iNOS is blocked by steroids such as dexamethasone. NOS is observed immunohistochemically in amygdala, grey matter, substantia gelatinosa of the spinal cord. These regions contain opioids receptors and are areas important in the control of the pain response. There is evidence from the literature that non-selective NOS inhibitors such as N^{G}-nitro arginine (NO₂Arg) prevents and reverses morphine-induced tolerance.

Taken together, these findings highlight the possible role of NO in the development of morphine-induced tolerance. Inhibition of NOS by non-selective NOS inhibitors such as NO₂Arg is associated with a vast array of detrimental side effects. To name but a few, these include: increased blood pressure and hypertension, increased platelet and white blood cell reactivity, decreased cerebral blood flow, gastrointestinal and renal toxicity.

Another problem with the use of opioids is severe physical dependence on the opioid which makes withdrawal very unpleasant. This makes it difficult for the clinician to take patients off the opioid when pain relief occurs or when an alternative route of pain relief is necessary because of the fear of eliciting unpleasant side effects originating from the abrupt removal of the opioid. Clonidine and buprenorphine are used in the clinic to attenuate the symptoms of withdrawal. However, these have their own side effects. For instance, clonidine causes hypotension and its effectiveness is limited by the susceptibility of the patient to this effect and buprenorphine has opioid agonist property which provides for potential abuse of the drug. It is known that non-selective NOS inhibitors such as NO₂Arg will prevent or relieve withdrawal symptoms associated with morphine withdrawal. (Bhargana, H.N., "Attenuation of Tolerance to and Physical Dependence on Morphine in the Rat by Inhibition of Nitric Oxide Synthase," *Gen. Pharmac*., 26, 1049-1053, 1995) Nevertheless, as stated above, these drugs have a number of serious side effects limiting their therapeutic uses.

It is desired to find an effective means to prevent tolerance to opioids in the treatment of pain and to relieve side effects of withdrawal without the adverse effects of known treatments.

### Summary of the Invention

The present invention is directed to the prevention of opioid tolerance in a human or animal subject. An effective amount of a suitable opioid and an effective amount of at least one iNOS inhibitor is administered to the subject to prevent tolerance to the opioid.

The present invention is also directed to the alternation of the symptoms of opioid withdrawal in a human or animal subject. An effective amount of at least one iNOS inhibitor is administered to the subject for a period of time effective to attenuate the symptoms upon opioid withdrawal.

The present invention is further directed to the prevention or elimination of hyperalgesia induced by opioid. An effective amount of at least one iNOS inhibitor is administered to the subject to eliminate or prevent hyperalgesia.

In addition, the present invention is directed to a pharmaceutical composition containing an opioid; an iNOS inhibitor, and a pharmaceutically acceptable carrier.

It is to be understood that both the foregoing general description and the following detailed description are exemplary and explanatory only and are not restrictive of the present invention as claimed.

### Brief Description ofthe Drawings

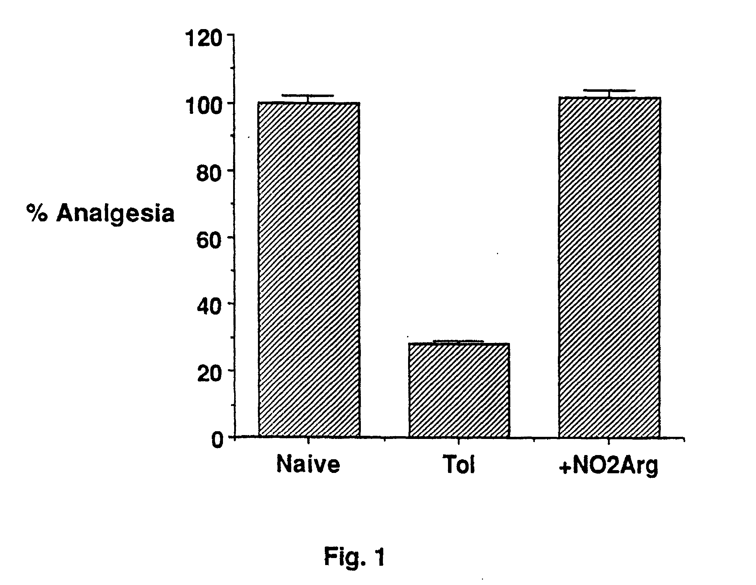

Fig. 1 depicts the inhibition of morphine-induced tolerance in mice by NO₂Arg.

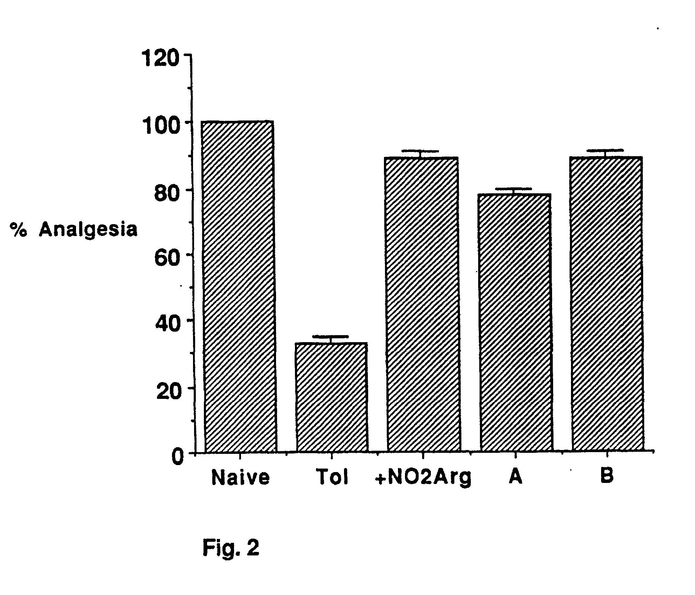

Fig. 2 depicts the inhibition of morphine-induced tolerance in mice by iNOS inhibitors.

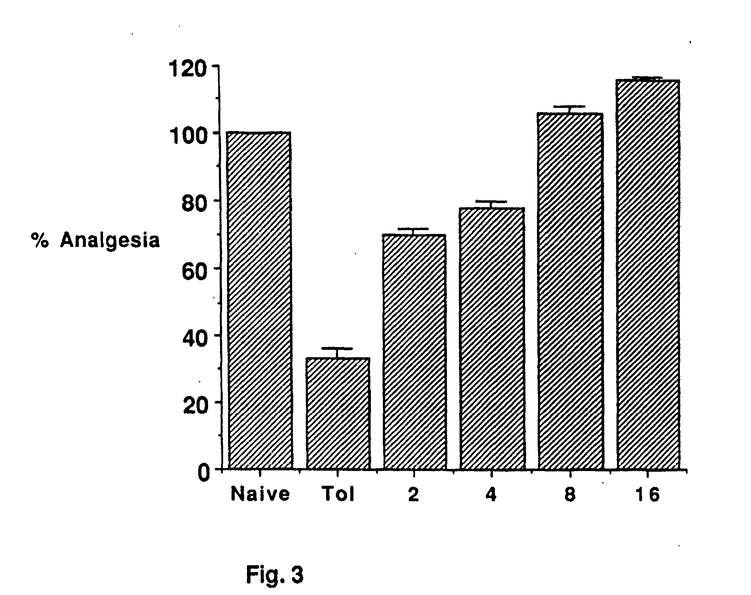

Fig. 3 depicts the inhibition of reduced analgesic effects of morphine in morphine-induced tolerant mice by an iNOS inhibitor.

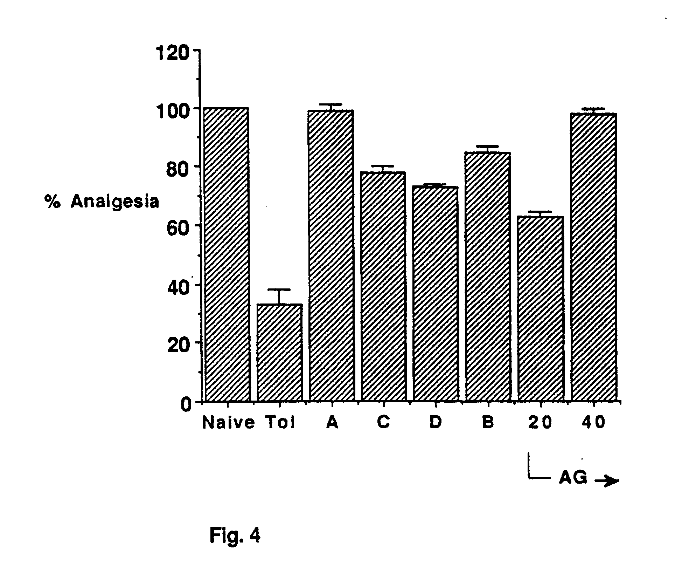

Fig. 4 depicts the prevention of loss of analgesic effects of morphine in morphine-tolerant mice by iNOS inhibitors.

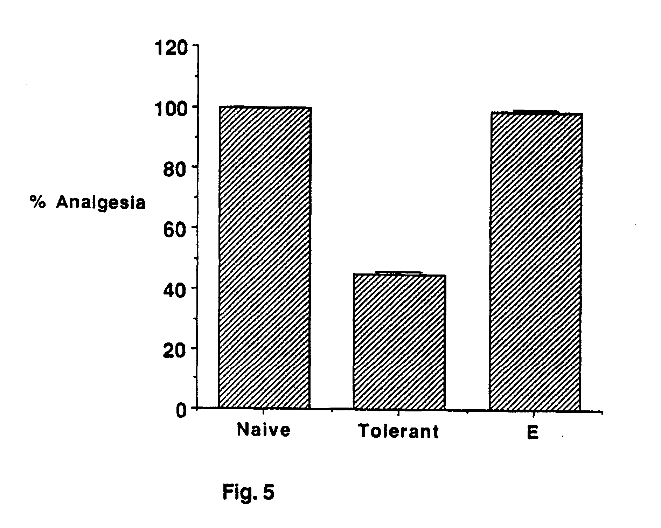

Fig. 5 depicts the effects of an iNOS inhibitor on morphine-induced tolerance in mice.

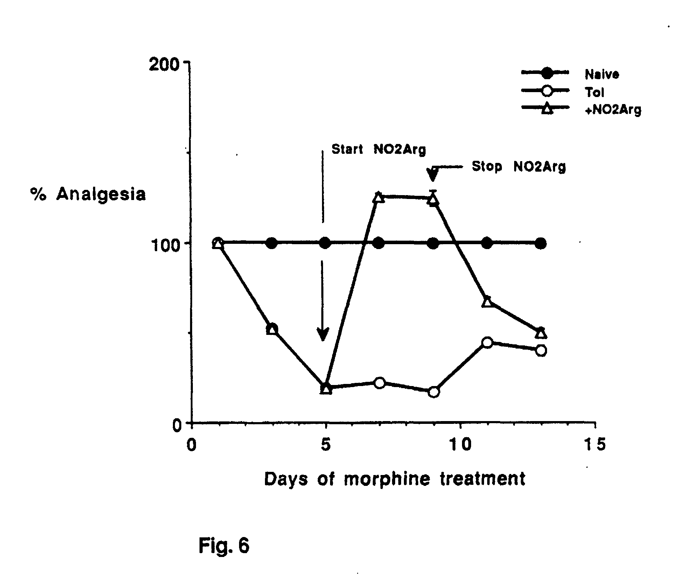

Fig. 6 depicts the reversal of morphine-induced tolerance by NO₂Arg.

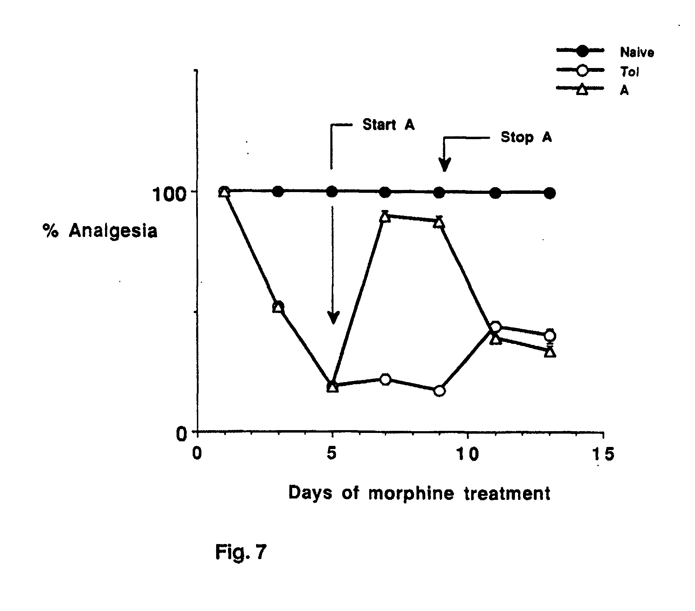

Fig. 7 depicts the reversal of morphine-induced tolerance by iNOS inhibition.
Fig. 8 depicts the acute effect of iNOS inhibition to reverse morphine tolerance (higher doses).
Fig. 9 depicts the acute effect of iNOS inhibition to reverse morphine tolerance (lower doses).

### Detailed Description of the Invention

The present invention relates to the use of iNOS inhibitors for the treatment and prevention of opioid tolerance. For instance, the dual administration of an iNOS inhibitor together with morphine in cancer patients allows lower doses of the morphine to elicit its analgesic effects while limiting its side effects. Moreover, an iNOS inhibitor can reverse opioid tolerance in patients that have already developed such tolerance. Thus the selective iNOS inhibitors restore the loss of analgesic effects observed upon prolonged treatment with an opioid. Furthermore, the selective inhibition of iNOS inhibitors do not have the side effects associated with non-selective NOS inhibitors.

The iNOS inhibitors themselves are not analgesic, nor do they potentiate the acute analgesic effects of morphine in non-tolerant animals. Acute administration of an iNOS inhibitor, once tolerance has developed, will restore the loss of analgesic effect of this opiate. The iNOS inhibitors at low doses attenuate opioid tolerance without affecting the antinocicoptive effects of morphine. This indicates selective action upon mechanisms involved with tolerance rather than simple potentiation of morphine. Thus in the cancer patient, if the iNOS inhibitor is administered with the morphine, there is no tolerance and analgesic effect is maintained. Consequently there is no need for an increase in morphine dose, there is reduced side effects, and there is an increase in clinical efficacy. In addition, iNOS inhibitors have no hemodynamic effect

Further, iNOS inhibitors will eliminate or prevent hyperalgesia associated with morphine use. Hyperalgesia is the enhanced nociceptive response to an otherwise non-noxious response and develops in association with the development of morphine tolerance. Both tolerance and hyperalgesia share an active, crucial role of activation of glutamatergic neurotransmission. Therefore, a subject treated with a narcotic may perceive more pain, in part due to the development of narcotic-induced hyperalgesia (NIH), in response to a preexisting painful condition even though there is no substantial increase in the intensity of peripheral factors causing pain. Thus progressively higher narcotic doses are needed to overcome the loss of analgesic effects of narcotics due to the development of both tolerance and NIH. In turn a vicious circle is initiated involving higher narcotic doses, more tolerance, greater NIH.

The use of an iNOS inhibitor will break the vicious circle and furthermore, will prevent hyperalgesia from occurring. Thus the present invention further relates to the use of iNOS inhibitors for the treatment and prevention of hyperalgesia.

The iNOS inhibitor of the present invention can be used with opioids in the treatment of many types of pain. Pain states include pain associated with tumor infiltration, e.g. bone and nerve, pain associated with cancer therapy, e.g. postsurgical syndromes, postchemotherapy syndromes, postradiation syndromes, neurophatic pain of diverse nature, acute postoperative pain, and acute pain associated with inflammatory response.

Based on data obtained, it is reasonable to expect that any iNOS inhibitor would work in the present invention. All iNOS inhibitors are envisioned to reduce or eliminate opioid tolerance and associated hyperalgesia, and to eliminate withdrawal symptoms. These iNOS inhibitors include those recited in related applications U.S. Serial Nos. 08/139,970, 08/141,168, 08/448,473, 08/635,188, 08/209,094, 08/218,160, 08/336,956, 08/425,831, and 08/438,321 and PCT applications 94/11724, 94/11832, 95/02669, 94/03589, 95/14001, 96/05315, and 96/06836. Also included are iNOS inhibitors disclosed in other works such as Merck & Co., Inc. including WO 96/18616, WO 96/14842, and WO 96/14842 and Abbott Laboratories including WO 94/12163.

Pharmaceutically acceptable salts, in particular acid addition salts, of the iNOS compounds are also contemplated for use in the present invention. Suitable salts include those formed with both organic and inorganic acids. Suitable salts are recognized in the Searle, Merck and Abbot applications.

The iNOS inhibitors useful in the present invention may be used alone, in combination with one another, or in combination with other classes of drugs useful in preventing tolerance to opioids or useful in attenuating the opioid withdrawal syndrome.

Preferably the iNOS inhibitor is administered as a pharmaceutical formulation containing one or more pharmaceutically acceptable carriers. The carriers should be compatible with the other ingredients of the formulation and not deleterious to the recipient thereof. Suitable carriers include, but are not limited to, distilled water, saline and acidified saline.

The formulations include those suitable for oral, parenteral (including subcutaneous, intradermal, intramuscular, intravenous, and intra articular), rectal and topical (including dermal, buccal, sublingual, and intraocular) administration. The formulations may conveniently be presented in unit dosage form and may be prepared by any of the methods well known in the art of pharmacy. Formulations of the present invention suitable for oral administration may be presented as discrete units such as capsules, cachets or tablets, each containing a predetermined amount of an active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary, or paste.

Formulations for parenteral administration include aqueous and non-aqueous sterile injection solutions which may contain antioxidants, buffers, bacteriostats and solutes which render the formulation isotonic with the blood of the intended recipient; and aqueous and nonaqueous sterile suspensions which may include suspending agents and thickening agents.

Formulations for rectal administration may be presented as a suppository with the usual carriers such as cocoa butter or polyethylene glycol. Formulations for topical administration in the mouth, for example buccally or sublingually, include lozenges and pastilles.

In addition to the ingredients particularly mentioned above, the formulations may include other agents conventional in the art.

Preferred unit dose formulations are those containing an effective dose, as hereinbelow recited, or an appropriate fraction thereof, of the active ingredient.

The iNOS inhibitors may be administered orally or via injection at a dose of from 0.1 to 300 mg/kg per day. The dose range for adult humans is generally from 5 mg to 500 mg/day. Tablets or other forms of presentation provided in discrete units may conveniently contain an amount of compound of the invention which is effective at such dosage or as a multiple of the same, for instance, units containing 5 mg to 500 mg, usually around 10 mg to 200 mg.

The amount of active ingredient that may be combined with the carrier materials to produce a single dosage form will vary depending upon the host treated and the particular mode of administration.

It will be understood that the specific dose level for any particular patient will depend upon a variety of factors including the activity of the specific compound employed, the age, body weight, general health, sex, diets, time of administration, rate of excretion, drug combination, and the severity of the particular disease undergoing therapy.

The iNOS inhibitors are preferably administered orally or by injection (intravenous or subcutaneous). The precise amount of the iNOS inhibitor administered to a patient will be the responsibility of the attending physician. However, the dose employed will depend on a number of factors, including the age and sex of the patient, and the particular opioid being used. The route of administration may also vary.

The iNOS compound can be administered with the opioid as a single bolus or in several doses. The dosage plan will depend on the factors discussed above, such as intensity of pain, age and sex of patient. A typical dosage may be twice a day.

### Examples

In the following examples, the following compounds were used as indicated.
Non-selective NOS inhibitor
   NO₂Arg N^{G}-monomethyl-L-arginine
Inducible NOS (iNOS) inhibitor

### Compound A

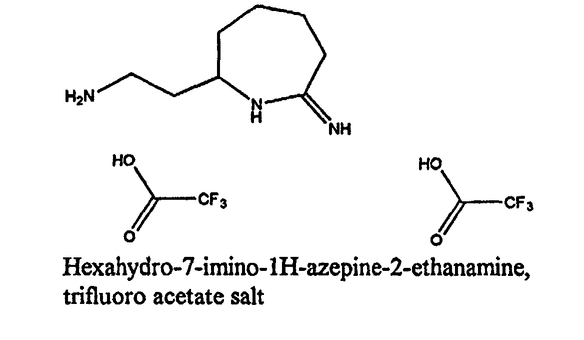

### Compound B

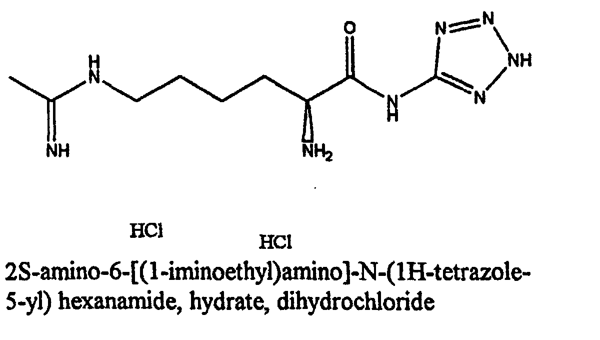

### Compound C

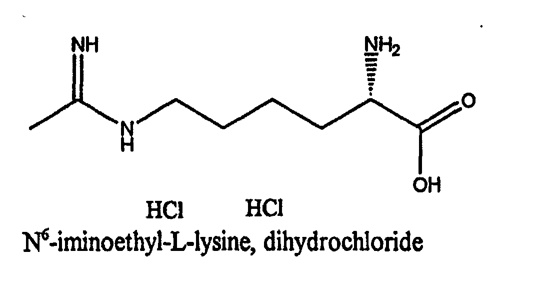

### Compound D

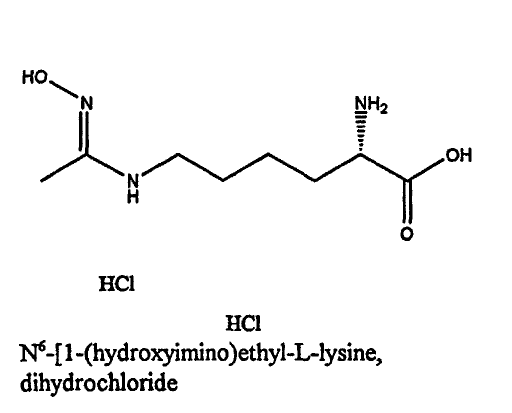

### Compound E

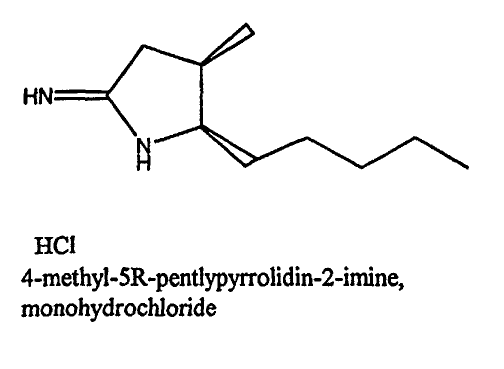

### Example 1

Mice were treated twice a day with either saline (naive) or morphine (s.c, 10 mg/kg) for a period of 4 days in order to induce tolerance (Tol). On day 5, all mice received a s.c. challenge dose of morphine (3 mg/kg) and analgesia measured 30 min. later. Dose response to morphine had indicated that a challenge dose of 3 mg/kg normally elicits a 90% analgesia in naive non-tolerant mice when assessed by standard hot plate test. As can be seen from this example, mice that were treated with morphine for the 4 days period responded with a lessened analgesic response to morphine when compared to the naive mice (this loss of analgesia is referred to as tolerance). Tolerance to morphine was restored in mice that were treated with the non-selective NOS inhibitor NO₂Arg. NO₂Arg was given intraperitoneally (i.p) twice a day at 4 mg/kg before each morphine injection. The total number of mice in each group was 20.

### Example 2

Mice were made tolerant to morphine as described in Example 1. One set of mice was treated with NO₂Arg (4 mg/kg i.p twice a day for 4 days before each sc morphine injection), the other two groups with the selective iNOS inhibitors Compounds A and B, (3 mg/kg i.p twice a day before each sc morphine injection). The iNOS inhibitors prevented morphine-induced tolerance. The total number of mice in each group was 20.

### Example 3

Mice were made tolerant to morphine as described in Example 1. Mice were treated with different doses of the selective iNOS inhibitor Compound A (1 to 8 mg/kg twice a day for 4 days with each dose given before each sc morphine injection). Compound A prevented in a dose-dependent manner the development of tolerance. The total number of mice in each group was 20.

### Example 4

Mice were made tolerant to morphine as described in Example 1. Shows inhibition of morphine induced tolerance to the analgesic effects of morphine with the iNOS inhibitors Compounds A, B, C, and D. The iNOS inhibitors were given i.p at 4 mg/kg twice a day (each dose before each morphine injection) for 4 days. Example also shows that much higher doses of the less selective iNOS inhibitors aminoguanidine (AG) was required to inhibit morphine induced tolerance (10 or 20 mg/kg a day twice a day for 4 days with each dose given before each morphine injection). The total number of mice in each group was 20.

### Example 5

Mice were made tolerant to morphine as described in Example 1. Shows inhibition of the reduced analgesic effects of morphine in morphine induced tolerant mice by an iNOS inhibitor Compound E. the iNOS inhibitor was given i.p at 4 mg/kg twice a day (each dose given before each morphine injection) for 4 days. The total number of mice in each group was 20.

### Example 6

Shows reversal by NO₂Arg of established loss of analgesia to morphine in mice made tolerant to morphine. Two groups of mice were made tolerant to morphine by s.c dosing of morphine at 10 mg/kg twice a day for a period of 13 days. Control mice received (instead of morphine) a twice daily injection of saline (referred to as naive). Analgesic response to the usual challenge dose of morphine (3 mg/kg) was measured every other day for the total period of study (13 days) in every mouse. At day 5, it is clear that all mice (except naive) were tolerant to the analgesic effects of morphine. At this time point, one of these groups received i.p injections of NO₂Arg (4 mg/kg twice a day with each dose given before each morphine injection) for the next 4 days. Analgesia was measured by hot plate every 2 days. It is clear that NO₂Arg reverses established tolerance to the analgesic effects of morphine. On day 9, the NO₂Arg was stopped and analgesia measured every 2 days until day 13. Reversal of tolerance by NO₂Arg is lost when the treatment was stopped. The total number of mice in each group was 20.

### Example 7

The iNOS inhibitor Compound A reversed the established loss of analgesia to morphine in mice made tolerant to morphine. The protocol and drug dosing regimen used in this example was identical to that described in Example 6. Compound A reverses established tolerance to the analgesic effects of morphine. Reversal of tolerance is lost upon removal of this selective iNOS inhibitor. The total number of mice in each group was 20.

### Example 8

This example shows the acute effect of iNOS inhibition to reverse morphine tolerance.

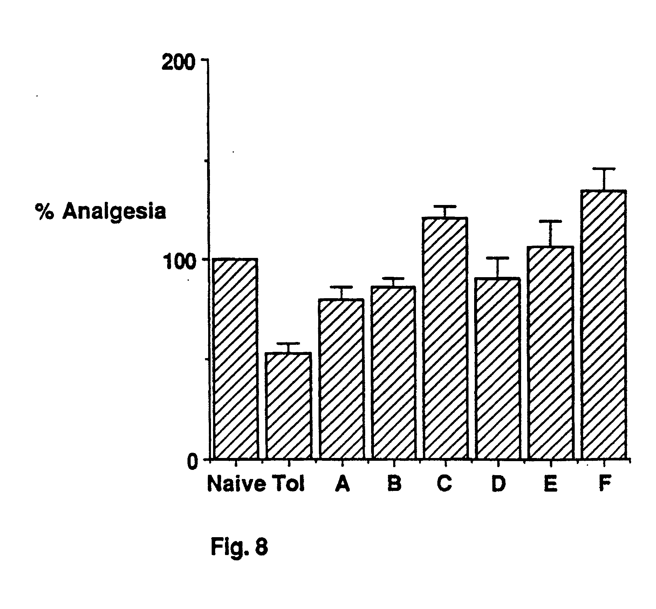

Part 1. Mice were treated twice a day with either saline (naive) or morphine (s.c., 10 mg/kg) for 12 days to induce and maintain tolerance. On days 5, 8, and 12, several doses (ip, mg/kg) of Compound E (A 0.4, B 1.0, C 4.0), Compound A (D 1.0, E 4.0) and NO₂Arg (F 4.0) were injected 40 min. before a s.c. challenge dose of 3.0 mg/kg morphine. % Analgesia was measured 50 min. later. The total number of mice in each group was 10-20. The results are shown Figure 8.

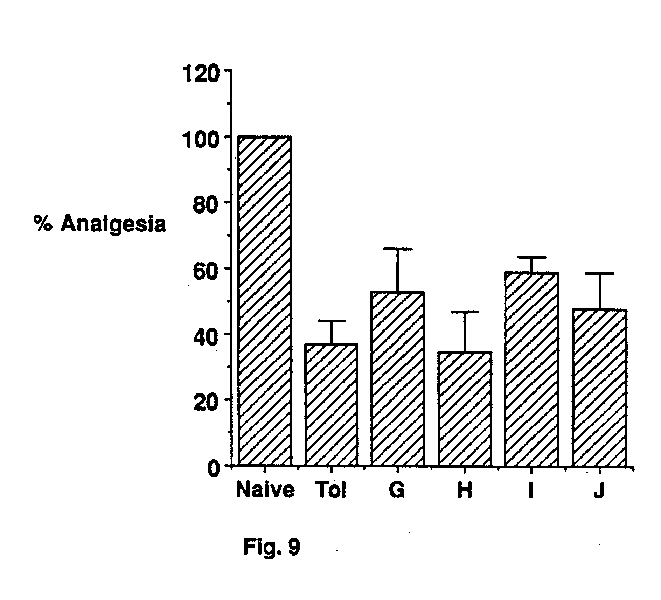

Part 2. Mice were treated in the same way as part 1 except on days 5, 8, and 12, lower acute doses (ip, mg/kg) of Compound E (G 0.04, H 0.1) and Compound A (I 0.04 J 0.1) were injected. The effect of Compounds E and A was dose dependent. The results are shown in Figure 9.

## Claims

1. Use of an effective amount of a suitable opioid and an effective amount of at least one selective iNOS inhibitor to prevent tolerance to the opioid for preparing a medicament for preventing opioid tolerance in a mammal.

2. The use of claim 1 wherein the opioid is morphine.

3. The use of claim 1 wherein the selective iNOS inhibitor is selected from the group consisting of hexahydro-7-imino-1H-azepine-2-ethanamine, 2S-amino-6-[(1-iminoethyl)amino]-N-(1H-tetrazole-5-yl) hexanamide; N⁶-iminoethyl-L-lysine, N⁶-[1-(hydroxyimino)ethyl-L-lysine, dihydrochloride, 4-methyl-5R-pentylpyrrolidin-2-imine, and pharmaceutically acceptable salts thereof.

4. The use of claim 1 in which said administering is conducted orally, intramuscularly, subcutaneously, transdermally, intravenously, or intra peritoneally.

5. The use of claim 1 wherein the seletive iNOS inhibitor is administered in a pharmaceutically acceptable carrier.

6. The use of claim 5 wherein the pharmaceutically acceptable carrier comprises distilled water, saline or acidified saline.

7. The use of claim 5 wherein the pharmaceutically acceptable carrier contains at least one selected from the group consisting of antioxidants, stabilizers, buffers, bacteriostats, suspending agents, and thickening agents.

8. The use of claim 1 wherein the effective amount of the selective iNOS inhibitor is about 0.1 to 300 mg/kg per day.

9. A use of an opioid withdrawal symptom attenuating amount of selective iNOS inhibitor for a period of time effective to attenuate said symptoms upon opioid withdrawal, said iNOS inhibitor being selected from the group consisting of hexahydro-7-imino-1H-azepine-2-ethanamine, 2S-amino-6-[(1-iminoethyl)amino]-N-(1H-tetrazole-5-yl) hexanamide, N⁶-iminoethyl-L-lysine, N⁶-[1-(hydroxyimino)ethyl-L-lysine, dihydrochloride, 4-methyl-5R-pentylpyrrolidin-2-imine, and pharmaceutically acceptable salts thereof for preparing a medicament for attenuating the symptoms of opioid withdrawal in a mammal.

10. The use of claim 9 wherein the opioid is morphine.

11. The use of claim 9 in which said administering is conducted orally, intramuscularly, subcutaneously, transdermally, intravenously, or intra peritoneally.

12. The use of claim 9 wherein the selective iNOS inhibitor is administered in a pharmaceutically acceptable carrier.

13. The use of claim 12 wherein the pharmaceutically acceptable carrier comprises distilled water, saline or acidified saline.

14. The use of claim 12 wherein the pharmaceutically acceptable carrier contains at least one selected from the group consisting of antioxidants, stabilizers, buffers, bacteriostats, suspending agents, and thickening agents.

15. The use of claim 9 wherein the effective amount of the selective iNOS inhibitor is 0.1 to 300 mg/kg per day.

16. A use of an effective amount of at least one selective iNOS inhibitor to eliminate or prevent hyperalgesia for preparing a medicament for preventing or eliminating hyperalgesia induced by opioid use, said iNOS inhibitor being selected from the group consisting of hexahydro-7-imino-1H-azepine-2-ethanamine, 2S-amino-6-[(1-iminoethyl)amino]-N-(1H-tetrazole-5-yl) hexanamide, N⁶-iminoethyl-L-lysine, N⁶-[1-(hydroxyimino)ethyl-L-lysine, dihydrochloride, 4-methyl-5R-pentylpyrrolidin-2-imine, and pharmaceutically acceptable salts thereof.

17. Use of an effective amount of at least one selective iNOS inhibitor to reverse tolerance to the opioid for preparing a medicament for reversing opioid tolerance in a mammal.

18. The use of claim 17 wherein the opioid is morphine.

19. The use of claim 17 wherein the selective iNOS inhibitor is selected from the group consisting of hexahydro-7-imino-1H-azepine-2-ethanamine, 2S-amino-6-[(1-iminoethyl)amino]-N-(1H-tetrazole-5-yl) hexanamide, N⁶-iminoethyl-L-lysine, N⁶-[1-(hydroxyimino)ethyl-L-lysine, dihydrochloride, 4-methyl-5R-pentylpyrrolidin-2-imine, and pharmaceutically acceptable salts thereof.

20. The use of claim 17 in which said administering is conducted orally, intramuscularly, subcutaneously, transdermally, intravenously, or intra peritoneally.

21. The use of claim 17 wherein the selective iNOS inhibitor is administered in a pharmaceutically acceptable carrier.

22. The use of claim 21 wherein the pharmaceutically acceptable carrier comprises distilled water, saline or acidified saline.

23. The use of claim 21 wherein the pharmaceutically acceptable carrier contains at least one selected from the group consisting of antioxidants, stabilizers, buffers, bacteriostats, suspending agents, and thickening agents.

24. The use of claim 17 wherein the effective amount of the selective iNOS inhibitor is 0.1 to 300 mg/kg per day.

25. A pharmaceutical composition comprising:
a) an opioid;
b) a selective iNOS inhibitor; and
c) a pharmaceutically acceptable carrier.

26. The composition of claim 25 wherein the opioid is morphine.

27. The composition of claim 25 wherein the selective iNOS inhibitor is selected from the group consisting of hexahydro-7-imino-1H-azepine-2-ethanamine, 2S-amino-6-[(1-iminoethyl)amino]-N-(1H-tetrazole-5-yl) hexanamide, N⁶-iminoethyl-L-lysine, N⁶-[1-(hydroxyimino)ethyl-L-lysine, dihydrochloride, 4-methyl-5R-pentylpyrrolidin-2-imine, and pharmaceutically acceptable salts thereof.

28. The composition of claim 25 wherein the pharmaceutically acceptable carrier comprises distilled water, saline or acidified saline.

29. The composition of claim 25 wherein the pharmaceutically acceptable carrier contains at least one selected from the group consisting of antioxidants, stabilizers, buffers, bacteriostats, suspending agents, and thickening agents.

## Patentansprüche

1. Verwendung einer wirksamen Menge eines geeigneten Opioids und einer wirksamen Menge von mindestens einem selektiven iSOS-Inhibitors zur Verhinderung der Toleranz gegenüber dem Opioid zur Herstellung eines Medikaments zur Verhinderung der Opioid-Toleranz bei einem Säuger.

2. Verwendung nach Anspruch 1, worin das Opioid Morphin ist.

3. Verwendung nach Anspruch 1, worin der selektive iSOS-Inhibitor aus der Gruppe bestehend aus Hexahydro-7-imino-1H-azepin-2-ethanamin, 2S-Amino-6-[(1-iminoethyl)amino]-N-(1H-tetrazol-5-yl)hexanamid, N⁶-Iminoethyl-L-lysin, N⁶-[1-(Hydroxyimino)ethyl-L-lysin, 4-Methyl-5R-pentylpyrrolidin-2-imin und pharmazeutisch annehmbaren Salzen davon ausgewählt ist.

4. Verwendung nach Anspruch 1, worin die Verabreichung oral, intramuskulär, subkutan, transdermal, intravenös oder intraperitoneal durchgeführt wird.

5. Verwendung nach Anspruch 1, worin der selektive iSOS-Inhibitor in einem pharmazeutisch annehmbaren Träger verabreicht wird.

6. Verwendung nach Anspruch 5, worin der pharmazeutisch annehmbare Träger destilliertes Wasser, Kochsalzlösung oder angesäuerte Kochsalzlösung umfasst.

7. Verwendung nach Anspruch 5, worin der pharmazeutisch annehmbare Träger mindestens einen aus der Gruppe bestehend aus Antioxidantien, Stabilisatoren, Puffern, Bakterostatika, Suspensions- und Verdickungsmitteln ausgewählten Bestandteil enthält.

8. Verwendung nach Anspruch 1, worin die wirksame Menge des ausgewählten iSOS-Inhibitors zwischen etwa 0,1 bis 300 mg/kg pro Tag liegt.

9. Verwendung einer Opioid-Entzugssymptome verbessernden Menge eines selektiven iSOS-Inhibitors über einen Zeitraum, der wirksam zur Verbesserung dieser Symptome bei Opioid-Entzug ist, wobei der iSOS-Inhibitor aus der Gruppe bestehend aus Hexahydro-7-imino-1H-azepin-2-ethanamin, 2S-Amino-6-[(1-iminoethyl)amino]-N-(1Htetrazol-5-yl)hexanamid, N⁶-Iminoethyl-L-lysin, N⁶-[1-(Hydroxyimino)ethyl-L-lysin, 4-Methyl-5R-pentylpyrrolidin-2-imin und pharmazeutisch annehmbaren Salzen davon ausgewählt ist, zur Herstellung eines Medikaments zur Verbesserung der Opioid-Entzugssymptome bei einem Säuger.

10. Verwendung nach Anspruch 9, worin das Opioid ein Morphin ist.

11. Verwendung nach Anspruch 9, worin die Verabreichung oral, intramuskulär, subkutan, transdermal, intravenös oder intraperitoneal durchgeführt wird.

12. Verwendung nach Anspruch 9, worin der selektive iSOS-Inhibitor in einem pharmazeutisch annehmbaren Träger verabreicht wird.

13. Verwendung nach Anspruch 12, worin der pharmazeutisch annehmbare Träger destilliertes Wasser, Kochsalzlösung oder angesäuerte Kochsalzlösung umfasst.

14. Verwendung von Anspruch 12, worin der pharmazeutisch annehmbare Träger mindestens einen aus der Gruppe bestehend aus Antioxidantien, Stabilisatoren, Puffern, Bakteriostatika, Suspensions- und Verdickungsmitteln ausgewählten Bestandteil enthält.

15. Verwendung nach Anspruch 9, worin die wirksame Menge des selektiven iSOS-Inhibitors 0,1 bis 300 mg/kg pro Tag ist.

16. Verwendung einer wirksamen Menge mindestens eines selektiven iSOS-Inhibitors zur Beseitigung oder Vorbeugung der Hyperalgesie zur Herstellung eines Medikaments zur Beseitigung oder Vorbeugung der durch Opioidgebrauch induzierten Hyperalgesie, wobei dieser iSOS-Inhibitor aus der Gruppe bestehend aus Hexahydro-7-imino-1Hazepin-2-ethanamin, 2S-Amino-6-[(1-iminoethyl)amino]-N-(1H-tetrazol-5-yl)hexanamid, N⁶-iminoethyl-L-lysin. N⁶-[1-(hydroxyimino)ethyl-L-lysin, 4-Methyl-5R-pentylpyrrolidin-2-imin und pharmazeutisch annehmbaren Salzen davon ausgewählt ist.

17. Verwendung einer wirksamen Menge mindestens eines selektiven iSOS-Inhibitors zur Umkehr der Toleranz gegenüber dem Opioid zur Herstellung eines Medikaments zur Umkehr der Opioid-Toleranz bei einem Säuger.

18. Verwendung nach Anspruch 17, worin das Opioid Morphin ist.

19. Verwendung nach Anspruch 17, worin der selektive iSOS-Inhibitor aus der Gruppe bestehend aus Hexahydro-7-imino-1H-azepin-2-ethanamin, 2S-Amino-6-[(1-iminoethyl)amino]-N-(1H-tetrazol-5-yl)hexanamid, N⁶-Iminoethyl-L-lysin, N⁶-[1-(Hydroxyimino)ethyl-L-lysin, 4-Methyl-5R-pentylpyrrolidin-2-imin und pharmazeutisch annehmbaren Salzen davon ausgewählt ist.

20. Verwendung nach Anspruch 17, worin die Herstellung oral, intramuskulär, subkutan, transdermal, intravenös oder intraperitoneal durchgeführt wird.

21. Verwendung nach Anspruch 17, worin der selektive iSOS-Inhibitor in einem pharmazeutisch annehmbaren Träger verabreicht wird.

22. Verwendung nach Anspruch 21, worin der pharmazeutisch annehmbare Träger destilliertes Wasser, Kochsalzlösung oder angesäuerte Kochsalzlösung umfasst.

23. Verwendung nach Anspruch 21, worin der pharmazeutisch annehmbare Träger mindestens einen aus der Gruppe bestehend aus Antioxidantien, Stabilisatoren, Puffern, Bakteriostatika, Suspensions- und Verdickungsmitteln ausgewählten Bestandteil enthält.

24. Verwendung nach Anspruch 17, worin die wirksame Menge des selektiven iSOS-Inhibitor zwischen 0,1 bis 300 mg/kg pro Tag beträgt.

25. Pharmazeutische Zusammensetzung umfassend
a) ein Opioid,
b) einen selektiven iSOS-Inhibitor und
c) einen pharmazeutisch annehmbaren Träger.

26. Zusammensetzung nach Anspruch 25, worin das Opioid Morphin ist.

27. Zusammensetzung nach Anspruch 25, worin der selektive iSOS-Inhibitor aus der Gruppe bestehend aus Hexahydro-7-imino-1H-azepin-2-ethanamin, 2S-Amino-6-[(1-iminoethyl)amino]-N-(1H-tetrazol-5-yl)hexanamid, N⁶-Iminoethyl-L-lysin, N⁶-[1-(hydroxyimino)ethyl-L-lysin, 4-Methyl-5R-pentylpyrrolidin-2-imin und pharmazeutisch annehmbaren Salzen davon ausgewählt ist.

28. Zusammensetzung von Anspruch 25, worin der pharmazeutisch annehmbare Träger destilliertes Wasser, Kochsalzlösung oder angesäuerte Kochsalzlösung umfasst.

29. Verbindung von Anspruch 25, worin der pharmazeutisch annehmbare Träger mindestens einen ausgewählten Bestandteil aus der Gruppe enthält, die aus Antioxidantien, Stabilisatoren, Puffern, Bakteriostatika, Suspensions- und Verdickungsmitteln besteht.

## Revendications

1. Utilisation d'une quantité efficace d'un opioïde convenable et d'une quantité efficace d'au moins un inhibiteur sélectif d'iNOS pour prévenir la tolérance aux opioïdes dans la préparation d'un médicament destiné à prévenir la tolérance aux opioïdes chez le mammifère.

2. Utilisation selon la revendication 1, dans laquelle l'opioïde est la morphine.

3. Utilisation selon la revendication 1, dans laquelle l'inhibiteur sélectif d'iNOS est choisi parmi l'ensemble constitué par l'hexahydro-7-imino-1H-azépine-2-éthanamine, le 2S-ainino-6-[(1-iminoéthyl)amino]-N-(1H-tétrazol-5-yl)hexanamide, la N⁶-iminoéthyl-L-lysine, la N⁶-[1-(hydroxyimino)éthyl]-L-lysine, la 4-méthyl-5R-pentylpyrrolidin-2-imine, et leurs sels pharmaceutiquement acceptables:

4. Utilisation selon la revendication 1, dans laquelle l'administration est effectuée par voie orale, intramusculaire, sous-cutanée, transdermique, intraveineuse, ou intrapéritonéale.

5. Utilisation selon la revendication 1, dans laquelle l'inhibiteur sélectif d'iNOS est administré dans un véhicule pharmaceutiquement acceptable.

6. Utilisation selon la revendication 5, dans laquelle le véhicule pharmaceutiquement acceptable comprend l'eau distillée, un sérum physiologique ou un sérum physiologique acidifié.

7. Utilisation selon la revendication 5, dans laquelle le véhicule pharmaceutiquement acceptable contient au moins un produit choisi parmi l'ensemble constitué par les antioxydants, stabilisants, tampons, agents bactériostatiques, agents de suspension, et épaississants.

8. Utilisation selon la revendication 1, dans laquelle la quantité efficace de l'inhibiteur sélectif d'iNOS est d'environ 0,1 à 300 mg/kg par jour.

9. Utilisation d'une quantité efficace d'un inhibiteur sélectif d'iNOS, atténuant les symptômes de privation d'opioïde pendant une période de temps, pour atténuer lesdits symptômes après sevrage d'opioïde, ledit inhibiteur sélectif d'iNOS étant choisi parmi l'ensemble constitué par l'hexahydro-7-imino-1H-azépine-2-éthanamine, le 2S-amino-6-[(1-iminoéthyl)-amino]-N-(1H-tétrazol-5-yl)hexanamide, la N⁶-iminoéthyl-L-lysine, la N⁶-[1-(hydroxyimino)éthyl]-L-lysine, la 4-méthyl-5R-pentylpyrrolidin-2-imine, et leurs sels pharmaceutiquement acceptables, dans la préparation d'un médicament destiné à atténuer les symptômes du sevrage d'opioïde chez le mammifère.

10. Utilisation selon la revendication 9, dans laquelle l'opioïde est la morphine.

11. Utilisation selon la revendication 9, dans laquelle l'administration est effectuée par voie orale, intramusculaire, sous-cutanée, transdermique, intraveineuse, ou intrapéritonéale.

12. Utilisation selon la revendication 9, dans laquelle l'inhibiteur sélectif d'iNOS est administré dans un véhicule pharmaceutiquement acceptable.

13. Utilisation selon la revendication 12, dans laquelle le véhicule pharmaceutiquement acceptable comprend l'eau distillée, un sérum physiologique ou un sérum physiologique acidifié.

14. Utilisation selon la revendication 12, dans laquelle le véhicule pharmaceutiquement acceptable contient au moins un produit choisi parmi l'ensemble constitué par les antioxydants, stabilisants, tampons, agents bactériostatiques, agents de suspension et épaississants.

15. Utilisation selon la revendication 9, dans laquelle la quantité efficace de l'inhibiteur sélectif d'iNOS est de 0,1 à 300 mg/kg par jour.

16. Utilisation d'une quantité efficace d'au moins d'un inhibiteur sélectif d'iNOS pour éliminer ou prévenir l'hyperalgésie dans la préparation d'un médicament destiné à prévenir ou à éliminer l'hyperalgésie induite par l'usage d'opioïde, ledit inhibiteur sélectif d'iNOS étant choisi parmi l'ensemble constitué par l'hexahydro-7-imino-1H-azépine-2-éthanamine, le 2S-amino-6-[(1-iminoéthyl)amino]-N-(1H-tétrazol-5-yl)hexanamide, la N⁶-iminoéthyl-L-lysine, la N⁶-[1-(hydroxyimino)éthyl]-L-lysine, la 4-méthyl-5R-pentylpyrrolidin-2-imine, et leurs sels pharmaceutiquement acceptables.

17. Utilisation d'une quantité efficace d'au moins un inhibiteur sélectif d'iNOS, pour inverser la tolérance aux opioïdes, dans la préparation d'un médicament destiné à inverser la tolérance aux opioïdes chez le mammifère.

18. Utilisation selon la revendication 17, dans laquelle l'opioïde est la morphine.

19. Utilisation selon la revendication 17, dans laquelle l'inhibiteur sélectif d'iNOS est choisi parmi l'ensemble constitué par l'hexahydro-7-imino-1H-azépine-2-éthanamine, le 2S-amino-6-[(1-iminoéthyl)amino]-N-(1H-tétrazol-5-yl)hexanamide, la N⁶-iminoéthyl-L-lysine, la N⁶-[1-(hydroxyimino)éthyl]-L-lysine, la 4-méthyl-5R-pentylpyrrolidin-2-imine, et leurs sels pharmaceutiquement acceptables.

20. Utilisation selon la revendication 17, dans laquelle l'administration est effectuée par voie orale, intramusculaire, sous-cutanée, transdermique, intraveineuse, ou intrapéritonéale.

21. Utilisation selon la revendication 17, dans laquelle l'inhibiteur sélectif d'iNOS est administré dans un véhicule pharmaceutiquement acceptable.

22. Utilisation selon la revendication 21, dans laquelle le véhicule pharmaceutiquement acceptable comprend l'eau distillée, un sérum physiologique ou un sérum physiologique acidifié.

23. Utilisation selon la revendication 21, dans laquelle le véhicule pharmaceutiquement acceptable contient au moins un produit choisi parmi l'ensemble constitué par les antioxydants, stabilisants, tampons, agents bactériostatiques, agents de suspension et épaississants.

24. Utilisation selon la revendication 17, dans laquelle la quantité efficace de l'inhibiteur sélectif d'iNOS est de 0,1 à 300 mg/kg par jour.

25. Composition pharmaceutique comprenant :
a) un opioïde ;
b) un inhibiteur séléctif d'iNOS ; et
c) un véhicule pharmaceutiquement.

26. Composition selon la revendication 25, dans laquelle l'opioide est la morphine.

27. Composition selon la revendication 25, dans laquelle l'inhibiteur sélectif d'iNOS est choisi parmi l'ensemble constitué par l'hexahydro-7-imino-1H-azépine-2-éthanamine, le 2S-amino-6-[(1-iminoéthyl)amino]-N-(1H-tétrazol-5-yl)hexanamide, la N⁶-iminoéthyl-L-lysine, la N⁶-[1-(hydroxyimino)éthyl]-L-lysine, la 4-méthyl-5R-pentylpyrrolidin-2-imine, et leurs sels pharmaceutiquement acceptables.

28. Composition selon la revendication 25, dans laquelle le véhicule pharmaceutiquement acceptable comprend l'eau distilléé, un sérum physiologique ou un sérum physiologique acidifié.

29. Composition selon la revendication 25, dans laquelle le véhicule pharmaceutiquement acceptable contient au moins un produit choisi parmi l'ensemble constitué par les antioxydants, stabilisants, tampons, agents bactériostatiques, des agents de suspension et épaississants.
